# EUROPEAN PATENT APPLICATION

(11) **EP 3 427 761 A2**
(43) Date of publication of application: **16.01.2019**
(21) Application number: 18183522.4
(22) Date of filing: 13.07.2018
(51) Int. Cl.: A61L 2/22, A62C 31/22

(54) **SYSTEM AND METHOD FOR MANAGING COMPLEX EMERGENCIES AND CONTAMINATED SITES**

(30) Priority: 14.07.2017 IT 201700079803
(71) Applicant: CRISTANINI S.P.A., 37010 Rivoli Veronese (VR) (IT)
(72) Inventor: Cristanini, Adolfo, 37010 Rivoli Veronese (VR) (IT)
(74) Representative: Bottino, Giovanni

(57) **Abstract**

A method for management of complex emergencies and contamination sites in order to recover a delimited area (1) affected by fire and industrial or pathogenic, chemical and/or biological and/or nuclear and/or radiological toxic agents, which area (1) is delimited by at least one boundary wall (2), the method comprising the following steps:
a) perforating (11) said boundary wall (2) by generating at least one hole;
b) extinguishing (12) the fire by dispensing a jet of a fire-extinguishing liquid through the hole;
c) decontaminating/detoxifying/disinfecting (13) the area (1) by dispensing a jet of a working fluid through the hole, said working fluid being supplemented with a decontamination/detoxification product.

## Description

The present invention relates to a method for management of complex emergencies and contamination sites in order to recover a delimited area affected by fire and industrial or pathogenic, chemical and/or biological and/or nuclear and/or radiological toxic agents, which area is delimited by at least one boundary wall. Thus, the method aims to eliminate or reduce the negative effects of aggressive CBRN (chemical, biological, radiological and nuclear) agents which are caused by natural disasters (including avalanches, landslides, etc.), industrial disasters and accidents, car accidents, war events or terrorist attacks, in interventions on hazardous materials (HAZMATs) or toxic industrial chemicals (TICs), and for decontamination/detoxification/disinfection purposes.

The term 'complex' refers to any emergency involving multiple events or events with multiple consequences and/or involving situations and substances which are dangerous to health and environment.

The boundary wall may be any obstacle, such as a wall of a reservoir; in this case, the aim may be to remove or drain its contents, whether they are solid, liquid or gaseous.

While decontamination and fire extinction belong to separate fields, they are intertwined very often with each other. Indeed, it is not unusual that an event requiring a decontamination intervention involves or triggers one or more fires that must be suppressed. Similarly, the development of a fire can result in the release of toxic pollutants or hazardous substances which have to be neutralized with a decontamination intervention in order to reduce their harmful effects on environment and human health. In both cases, it is necessary first to extinguish the fire and then carry out the decontamination.

Currently, these two operations are performed separately with different methods and apparatuses and often by different operators. This can lead to situations in which a great amount of time elapses from fire extinction to decontamination, thereby increasing both the risk for all the subjects and/or operators involved in the event and the risk for an uncontrolled spreading of pollutants throughout the environment.

Document EP1930083A2 by the same Applicant discloses a jet lance for application of fluids and/or decontamination/detoxification products, the lance comprising means for supplying a decontamination/detoxification product designed to be mixed with a working fluid and to be dispensed through a dispensing nozzle.

Document WO2007/032722A1 discloses an equipment for making holes into walls for extinguishing fires, the equipment comprising a source of a pressurized fire-extinguishing liquid and a lance provided with a fire-extinguishing nozzle and connected to the source through a tube. The fire-extinguishing liquid is dispensed to cut the wall in order to generate the hole; once the hole is formed, the fire-extinguishing liquid is injected into the burning area to extinguish the fire. The fire-extinguishing liquid is mixed with an additive to improve the cutting operation and/or the extinguishing effect.

Document DE202017102091U1 by the same Applicant discloses a lance for extinguishing a fire in a room. The lance comprises two channels, respectively for a pressurized fluid and an abrasive powder, and mixing means to mix the pressurized fluid and the abrasive powder by the Venturi effect. The pressurized fluid-abrasive powder mixture is used to make a hole into a wall of the room through which the pressurized fluid is dispensed to extinguish the fire.

Each of these documents currently known in the art discloses either fire-extinction methods and systems or decontamination/detoxification/disinfection methods and systems, thus confirming what described immediately before regarding the separate approaches employed to implement these two types of operations and systems as taught in the art.

Therefore, there is still an unsatisfied need for a method for management of complex emergencies and sites which are contaminated, potentially contaminated or at risk of being contaminated by the event, such a method comprising a series of fire-extinction-related steps and a series of decontamination/detoxification/disinfection-related steps.

The present invention aims to overcome the aforementioned disadvantages of the currently known methods by providing a method as described at the beginning of the present disclosure and further comprising the following steps:
a) perforating said boundary wall by generating at least one hole;
b) extinguishing the fire by dispensing a jet of a fire-extinguishing liquid through the hole;
c) decontaminating/detoxifying/disinfecting the area by dispensing a jet of a working liquid through the hole, said working liquid being supplemented with a decontamination/detoxification product.

The boundary wall can be variable in thickness and made of compact and non-compact materials. The hole may be ducted or jacketed in order to allow the system to pass through incongruent walls of considerable thickness as well as to allow the cutting liquid and any waste material to be drained out. Similarly, the tubing could be useful to drain hazardous fluids; if necessary, the perforation/spray system can be provided with rigid or flexible rods made of tubing.

Thus, the method for management of complex emergencies and contamination sites comprises first intervention (first responder) stages and event management (hazard management) stages, particularly, it comprises a fire-extinction step to extinguish a fire by forming a hole into the boundary wall, and a decontamination/detoxification/disinfection step. This results in a prompt intervention which can ensure a great effectiveness in totally securing the delimited area by managing both the fire and any contamination in a short time. The method also allows the operator to act immediately in the area in which the fire has developed from the outside. Therefore, the entire operation can be carried out by a single operator who, acting only through the hole made into the wall, is not subjected to the risk of being exposed to fumes and/or toxic vapours or back-drafts which commonly occur due to a sudden inflow of oxygen into the area affected by the fire when doors are opened or windows are broken. The method is particularly advantageous for use in potentially explosive areas. Accordingly, one of the advantages of the method is a high safety standard both for operators and for environment.

The decontamination and/or detoxification and/or disinfection step necessarily involves the use of a suitable decontamination/detoxification product to be added to the working fluid, and it cannot be considered as performed in an effective manner - even implicitly - with the simple application of the working fluid, whether it is plain water or a foam or a fluid supplemented with an abrasive powder. There are also HAZMAT cases in which plain water has not a decontamination/detoxification effect, but rather it increases the risks.

In a preferred exemplary embodiment, the decontamination/detoxification/disinfection step comprises a first stage in which a pre-wash is performed, a second stage in which the decontamination/detoxification product is applied, and a third stage in which a rinse is performed.

The decontamination/detoxification product can be of any type currently known in the art, but it is preferably a powder.

In one exemplary embodiment, the perforation step is carried out by a water-jet cutting operation which comprises applying a flow of a pressurized cutting liquid into the region of the boundary wall to be perforated.

More preferably, the cutting liquid consists of water and dispersed abrasive particles.

This allows a hole to be formed in a short time and water to be used as both the cutting liquid and the fire-extinguishing liquid.

According to one embodiment, the jet of the fire-extinguishing liquid is dispensed as finely dispersed drops.

More preferably, the fire-extinguishing liquid consists of water or water in admixture with a fire-extinguishing product. The fire-extinguishing product can be of any type currently known in the art, but it is preferably a foam.

The water is dispensed in an atomized form to greatly increase the contact surface between water and air, thereby absorbing a high amount of heat. This can subtract energy from the fire by a cooling effect which reduces the pressure within the area and therefore increases the diffusion of the atomized water. When in the form of finely dispersed drops, water can also reduce oxygen concentration: during its phase transition from liquid to vapor, water greatly increases in volume, thereby reducing the concentration of oxygen serving as a comburent for the fire.

In a further exemplary embodiment, the perforation step is preceded by a step of identifying the optimal area from which to attack the fire by thermal detection.

This can improve the effectiveness by attacking the fire from the more suitable point.

In one exemplary embodiment, the perforation, fire-extinction and decontamination/detoxification/disinfection steps are performed with the use of a single lance connected to a supply line for a pressurized liquid.

In a further exemplary embodiment, the lance is connected to a reservoir containing abrasive particles to be dispersed into the liquid in order to perform a water-jet cutting operation by applying a flow of the pressurized cutting liquid into the region of the boundary wall to be perforated.

In a further exemplary embodiment, the lance is provided with an atomizing nozzle for dispensing the liquid as finely dispersed drops, which liquid consists of water or water in admixture with a fire-extinguishing product.

In a further exemplary embodiment, the lance is connected to a reservoir for the decontamination/detoxification product to be mixed into the liquid.

Another object of the present invention is a system for management of complex emergencies and contamination sites in order to recover a delimited area affected by fire and industrial or pathogenic, chemical and/or biological and/or nuclear and/or radiological toxic agents, which area is delimited by at least one boundary wall, said system comprising means for perforating said boundary wall, said perforation means being adapted to generate at least one hole, fire-extinction means adapted to dispense a jet of a fire-extinguishing liquid through the hole, and means for decontaminating/detoxifying/disinfecting the area, said decontamination/detoxification/disinfection means being adapted to dispense a jet of a working fluid through the hole, said working fluid being supplemented with a decontamination/detoxification product.

In one exemplary embodiment, the perforation means comprise a lance connected to a supply line for a pressurized cutting liquid and to a reservoir for abrasive particles to be dispersed into the cutting liquid in order to perform a water-jet cutting operation which comprises applying a flow of the pressurized cutting liquid into the region of the boundary wall to be perforated. The lance can be provided with extensions and/or stands in order to reach regions which are difficult to be accessed.

In a further exemplary embodiment, the fire-extinction means comprise a lance which is connected to a supply line for the pressurized fire-extinguishing liquid and which is provided with an atomizing nozzle for dispensing the fire-extinguishing liquid as finely dispersed drops, which fire-extinguishing liquid consists of water or water in admixture with a fire-extinguishing product.

In one embodiment, the decontamination/detoxification/disinfection means comprise a lance connected to a supply line for the pressurized working fluid and to a reservoir for the decontamination/detoxification product in order to disperse the decontamination/detoxification product into the working liquid.

According to a further embodiment, a single lance and a single supply line are provided which can be alternately used as the perforation means, fire-extinction means and decontamination/detoxification/disinfection means.

This allows the above-described method to be carried out with the use of a compact and inexpensive system which can be employed by a single operator in an extremely fast way due to the absence of downtime for changing the tool to be used. Therefore, the perforation, fire-extinction and decontamination/detoxification/disinfection steps can be carried out with the use of a single system comprising a single lance, the latter step preferably comprising a first stage in which a pre-wash is performed, a second stage in which the decontamination/detoxification product is applied, and a third stage in which a rinse is performed.

In an advantageous exemplary embodiment, the lance is provided with means for switching from a perforation/fire-extinction configuration to a decontamination/detoxification/disinfection configuration, which means can be operated by a lever movable by the user.

More preferably, the means for switching from the perforation/fire-extinction configuration to the decontamination/detoxification/disinfection configuration are adjusted so as to switch from one configuration to the other without stopping the operations.

In one embodiment, the means for switching from a perforation/fire-extinction configuration to a decontamination/detoxification/disinfection configuration comprise a pressure reducer to reduce the pressure from a first, typically higher perforation/fire-extinction value to a second, typically lower decontamination/detoxification/disinfection value.

In a further embodiment, when in the decontamination/detoxification/disinfection configuration, the lance atomizes the liquid supplemented with the decontamination/detoxification product. This improves the solubilization of the product into the liquid, thereby increasing the effectiveness of the decontamination/detoxification/disinfection step. Moreover, in the presence of a radioactive contamination potentially resulting in a fallout, atomization decreases the volatilization of the radioactive particulate by binding thereto and precipitating it in situ, thereby preventing a secondary contamination from occurring in the surrounding environment.

In a first variant embodiment, two interchangeable reservoirs are provided, respectively for the abrasive particles and for the decontamination/detoxification product, in such a way that the user can use the lance connected to the reservoir containing the abrasive particles during the perforation/extinction steps, remove the reservoir containing the abrasive particles and substitute it with a reservoir containing the decontamination/detoxification product, and proceed with the decontamination/detoxification/disinfection step.

In a second variant embodiment, two separate reservoirs are provided which are connected in parallel to the supply line, and the means for switching from a perforation/fire-extinction configuration to a decontamination/detoxification/disinfection configuration comprise a deflector which allows the two reservoirs to be alternately connected to the supply line for the pressurized liquid.

In a third variant embodiment, a single reservoir is provided which is connected to the lance and which contains both the abrasive particles and the decontamination/detoxification product.

Alternatively, a first lance and a corresponding first supply line can be used as the perforation means and fire-extinction means, and a second lance and a corresponding second supply line can be used as the decontamination/detoxification/disinfection means. In a further alternative configuration, a first lance and a corresponding first supply line are provided which can be used as the perforation means, and a second lance and a corresponding second supply line are provided which can be used as the fire-extinction means and decontamination/detoxification/disinfection means.

According to an exemplary embodiment, thermal detection means are provided to identify the optimal area from which to attack the fire.

These and other features and advantages of the present invention will appear more clearly from the following description of certain embodiments illustrated in the accompanying drawings, wherein:
Fig. 1 shows a functional diagram of one exemplary embodiment of the method;
Fig. 2 shows an exemplary embodiment of the system;
Figs. 3 and 4 show exemplary embodiments of the system in which a single lance and a single supply line are provided.

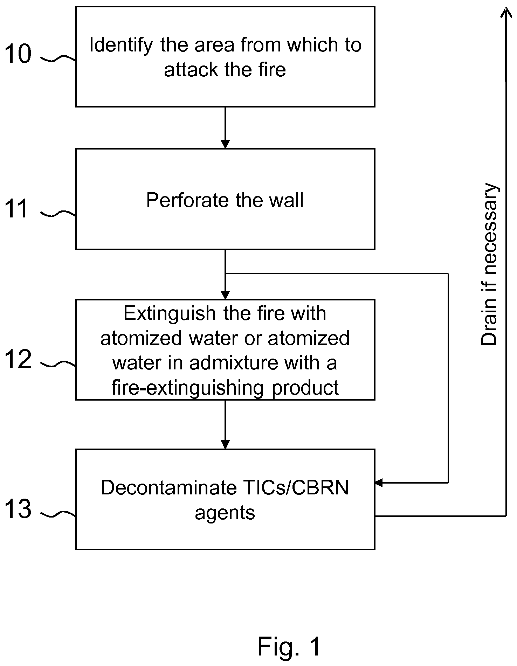

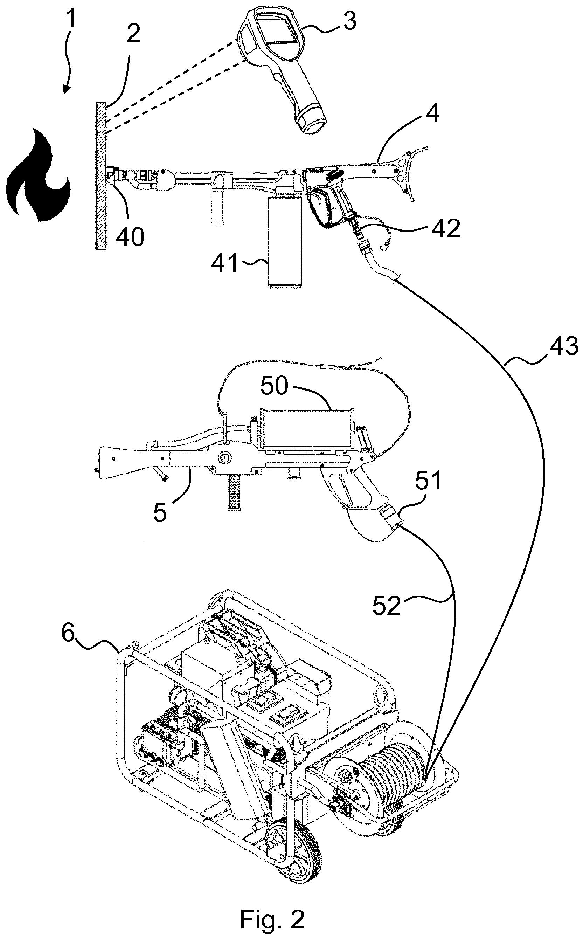

As schematically shown in Figure 1, the method of the present invention for management of complex emergencies and contamination sites aims to recover a delimited area 1 as visible in Figure 2, which area 1 is affected by fire and industrial or pathogenic, chemical and/or biological and/or nuclear and/or radiological toxic agents. The area 1 is delimited by at least one boundary wall 2.

The method can start with an optional step 10 of identifying the optimal area from which to attack the fire by thermal detection. The operator performs a thermal scan of the boundary wall 2 and obtains information about the fire beyond the wall 2 based on the detected thermal values. This allows the operator to choose the point from which to attack the fire in order to improve the effectiveness of the intervention.

Successively, the method contemplates a step 11 of perforating said boundary wall 1 by generating a hole. The hole is a through-hole which can establish a communication between the area in which the operator is located and the area 1 in which the fire has developed. The perforation step 11 is preferably carried out by a water-jet cutting operation which comprises applying a flow of a pressurized cutting liquid to the region of the boundary wall 2 to be perforated. Advantageously, the region of the hole is the region as determined at the identification step 10. Preferably, the cutting liquid consists of water and dispersed abrasive particles.

Thereafter, the method contemplates a fire-extinction step 12 which comprises dispensing a jet of a fire-extinguishing liquid through the hole. The jet of the fire-extinguishing liquid is preferably dispensed as finely dispersed drops. The fire-extinguishing liquid can consist of water or water in admixture with a fire-extinguishing product.

Successively, the method contemplates a step 13 of decontaminating/detoxifying/disinfecting the area 1 by dispensing a jet of a working fluid through the hole, said working fluid being supplemented with a decontamination/detoxification product.

The working fluid may comprise fresh water, salt water or other solvents known to those skilled in the art as a solvent.

If there is no fire or the fire has been already extinguished, it is possible to proceed directly from the wall-perforation step 11 to the TICs/CBRN-decontamination step 13.

If the contents of the delimited area 1 have to be removed or drained, whether they are solid, liquid or gaseous, the decontamination step can be followed by a drain step.

Figure 2 shows an exemplary embodiment of the system of the present invention for management of complex emergencies and contamination sites, said system being designed to implement the above-described method.

The system comprises means for perforating said boundary wall 2, said perforation means being adapted to generate at least one hole, fire-extinction means adapted to dispense a jet of a fire-extinguishing liquid through the hole, and means for decontaminating/detoxifying/disinfecting the area 1, said decontamination/detoxification/disinfection means being adapted to dispense a jet of a working fluid through the hole, said working fluid being supplemented with a decontamination/detoxification product.

In the example shown in the figure, the perforation means and the fire-extinction means comprise a single perforating/fire-extinguishing lance 4 which is fluidly connected to a supply line 43 for a pressurized cutting/fire-extinguishing fluid through a manifold 42. To this aim, for example, it is possible to use a WJFE® lance marketed by the same Applicant and described in Italian patent application VI2014A000150 in the name of the Applicant.

The lance 4 is designed to be grasped and directed by an operator, and it is provided with at least two nozzles 40 which can be selectively mounted to the lance 4 for different purposes.

As shown in the figure, the supply line 43 comprises an autonomous multi-purpose unit 6, for example a Sanijet® C-921 device marketed by the same Applicant and described in Italian patent applications VI2010A000221, VI2011A000070 and VI2011A000212 in the name of the Applicant, or a WJFE® device marketed by the same Applicant.

As a reference, the pressurized fluid may have a pressure between 90 bar and 650 bar.

The lance 4 comprises at least one duct for the pressurized fluid and a reservoir 41 containing an abrasive powder and fluidly connected to the duct, means being provided for mixing the abrasive powder into the pressurized fluid by the Venturi effect. For example, the abrasive powder may be a naturally-occurring material, such as GARNET@, or a synthetic material, such as SAMGRIT®, both having a particle size from 0.15 mm to 2.5 mm.

The lance 4 comprises selection means which allow the operator either to supply the abrasive powder into the pressurized fluid or alternatively to prevent the abrasive powder from being supplied into the pressurized fluid without needing to stop the operation of the lance 4.

This allows a water-jet cutting operation to be performed by dispensing the pressurized fluid supplemented with the abrasive powder into the region of the boundary wall to be perforated. The pressurized fluid may be water or water in admixture with a fire-extinguishing product.

It is possible to use a first nozzle for performing the water-jet cutting operation, and a second atomizing nozzle for dispensing the fire-extinguishing liquid as finely dispersed drops.

Alternatively, it is possible to form the hole with the use of a special drill, for example an ATEX (ATmospheres & EXplosibles) drill, in order to prevent the occurrence of explosions, and then extinguish the fire by dispensing the fire-extinguishing liquid atomized through the hole with the use of a special fire-extinguishing lance such as, for example, a Fire Stop lance marketed by the same Applicant and described in Italian patent application VI2012A000326 in the name of the Applicant. Such a lance allows the dispensed flows to be to accurately and constantly adjusted and the particles of fire-extinguishing liquid to be optimally atomized and dispersed.

The decontamination/detoxification/disinfection means shown in the figure comprise a lance 5 which is connected to a supply line 52 for the pressurized working fluid through a manifold 51.

For example, the lance 5 can be a Sanijetgun® lance marketed by the same Applicant and described in Italian patent application VI2006A000350 in the name of the Applicant.

As shown in the figure, the supply line 52 also comprises an autonomous multi-purpose unit 6 which can be the same as described before with reference to the supply line 43.

The lance 5 is provided with a duct for the working fluid and means for supplying the decontamination/detoxification product to the working fluid, said means being generally referred to by numeral 50 in the figure. Such means essentially comprise a cartridge for the product which can be removably coupled to the body of the lance, a flexible duct to disperse the decontamination/detoxification product into the working liquid, and a Venturi tube-type ejector positioned on the duct in order to connect it to the cartridge.

When a control lever is operated, the working fluid flows through the Venturi tube-type ejector, thereby creating a vacuum which draws the decontamination/detoxification product from the cartridge by the Venturi effect. The product is mixed with the working fluid, and the resulting mixture is conveyed to the nozzle in order to be dispensed into the area to be recovered.

For example, the decontamination/detoxification product can be the BX24® product marketed by the same Applicant and described in Italian patent application VI2008A000049 in the name of the Applicant.

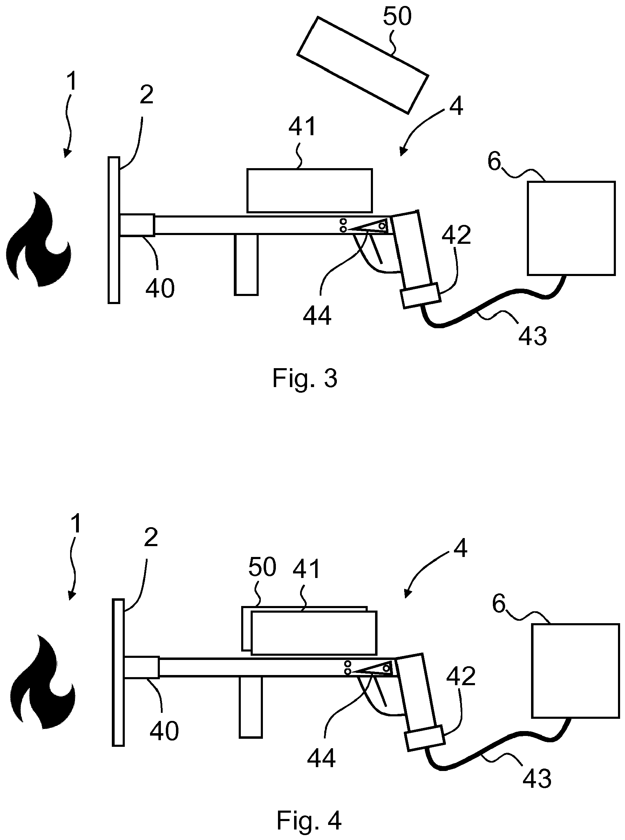

Figures 3 and 4 show exemplary embodiments of the system comprising a single lance 4 and a single supply line 43 which can be alternately used as the perforation means, fire-extinction means and decontamination/detoxification/disinfection means. The lance 4 is fluidly connected to the supply line 43 for the pressurized liquid through the manifold 42, the supply line 43 comprising an autonomous multi-purpose unit 6.

The lance 4 is provided with means for switching from a perforation/fire-extinction configuration to a decontamination/detoxification/disinfection configuration, which means can be operated by a lever 44 movable by the user. The lever 44 is movable between two positions, a first position associated with the perforation/fire-extinction configuration and a second position associated with the decontamination/detoxification/disinfection configuration. The means for switching from the perforation/fire-extinction configuration to the decontaminating/detoxifying/disinfection configuration are adjusted so as to switch from one configuration to the other one without stopping the operations. The means for switching from a perforation/fire-extinction configuration to a decontamination/detoxification/disinfection configuration comprise a pressure reducer to reduce the pressure from a first, typically higher perforation/fire-extinction value to a second, typically lower decontamination/detoxification/disinfection value.

The lance 4 is provided with an atomizing nozzle 40 in such a way that, when in the decontamination/detoxification/disinfection configuration, the lance can atomize the liquid supplemented with the decontamination/detoxification product.

The variant embodiment of Figure 3 comprises two interchangeable reservoirs, respectively a reservoir 41 for the abrasive particles and means 50 for supplying the decontamination/detoxification product to the pressurized liquid. The user can use the lance connected to the reservoir 41 containing the abrasive particles during the perforation/extinction steps, remove the reservoir 41 containing the abrasive particles and substitute it with the reservoir 50 containing the decontamination/detoxification product, and proceed with the decontamination/detoxification/disinfection step.

Alternatively, a single reservoir 41 can be provided which is connected to the lance 4 and which contains both the abrasive particles and the decontamination/detoxification product in admixture with each other.

In the variant embodiment of Figure 4, two separate reservoirs are provided which are connected in parallel to the supply line 43, respectively a reservoir 41 for the abrasive particles and means 50 for supplying the decontamination/detoxification product to the pressurized liquid. In this case, the means for switching from a perforation/fire-extinction configuration to a decontamination/detoxification/disinfection configuration comprise a deflector which allows the two reservoirs 41 and 50 to be alternately connected to the supply line 43 for the pressurized liquid.

Figure 4 shows the two reservoirs 41 and 50 as mounted to the lance 4; however, at least one or both of the reservoirs can be mounted to a suitable technical chest harness in many possible positions, for example by means of multiple anchors provided on the chest harness.

## Claims

1. A method for management of complex emergencies and contamination sites in order to recover a delimited area (1) affected by fire and industrial or pathogenic, chemical and/or biological and/or nuclear and/or radiological toxic agents, which area is delimited by at least one boundary wall (2),
**characterized in that**
it comprises the following steps:
a) perforating (11) said boundary wall (2) by generating at least one hole;
b) extinguishing (12) the fire by dispensing a jet of a fire-extinguishing liquid through the hole;
c) decontaminating/detoxifying/disinfecting (13) the area by dispensing a jet of a working fluid through the hole, said working fluid being supplemented with a decontamination/detoxification product.

2. The method according to claim 1, wherein the perforation step (11) is carried out by a water-jet cutting operation which comprises applying a flow of a pressurized cutting liquid to the region of the boundary wall (2) to be perforated.

3. The method according to claim 2, wherein the cutting liquid consists of water and dispersed abrasive particles.

4. The method according to one or more of the preceding claims, wherein the jet of fire-extinguishing liquid is delivered as finely dispersed drops.

5. The method according to one or more of the preceding claims, wherein the fire-extinguishing liquid consists of water or water in admixture with a fire-extinguishing product.

6. The method according to one or more of the preceding claims, wherein the perforation step (11) is preceded by a step (10) of identifying the optimal area from which to attack the fire by thermal detection.

7. The method according to one or more of the preceding claims, wherein the perforation step (11), the fire-extinction step (12) and the decontamination/detoxification/disinfection step (13) are carried out by using a single lance (4) connected to a supply line (43) for a pressurized liquid.

8. The method according to claim 7, wherein the lance (4) is connected to a reservoir (41) containing abrasive particles to be dispersed into the liquid to perform a water-jet cutting operation by applying a flow of the pressurized cutting liquid to the region of the boundary wall (2) to be perforated.

9. The method according to claim 7 or 8, wherein the lance (4) is provided with an atomizing nozzle (40) for dispensing the liquid as finely dispersed drops, which liquid consists of water or water in admixture with a fire-extinguishing product.

10. The method according to one or more of claims 7 to 9, wherein the lance (4) is connected to a reservoir containing a decontamination/detoxification product to be mixed into the liquid.

11. A system for management of complex emergencies and contamination sites in order to recover a delimited area (1) affected by fire and industrial or pathogenic, chemical and/or biological and/or nuclear and/or radiological toxic agents, which area is delimited by at least one boundary wall (2),
**characterized in that**
it comprises means for perforating said boundary wall (2), said perforation means being adapted to generate at least one hole, fire-extinction means adapted to dispense a jet of a fire-extinguishing liquid through the hole, and means for decontaminating/detoxifying/disinfecting the area (1), said decontamination/detoxification/disinfection means being adapted to dispense a jet of a working fluid through the hole, said working fluid being supplemented with a decontamination/detoxification product.

12. The system according to claim 11, wherein the perforation means comprise a lance (4) connected to a supply line (43) for a pressurized cutting liquid, and a reservoir (41) containing abrasive particles to be dispersed into the cutting liquid to perform a water-jet cutting operation by applying a flow of the pressurized cutting liquid to the region of the boundary wall (2) to be perforated.

13. The system according to claim 11 or 12, wherein the fire-extinction means comprise a lance (4) which is connected to a supply line (43) for the pressurized fire-extinguishing liquid and which is provided with an atomizing nozzle (40) for dispensing the fire-extinguishing liquid as finely dispersed drops, which fire-extinguishing liquid consists of water or water in admixture with a fire-extinguishing product.

14. The system according to one or more of claims 11 to 13, wherein a single lance and a single supply line are provided which can be alternately used as the perforation means, fire-extinction means and decontamination/detoxification/disinfection means.

15. The system according to one or more of claims 11 to 14, wherein thermal detection means (3) are provided for identifying the optimal zone from which to attack the fire.
